# EUROPEAN PATENT APPLICATION

(11) **EP 4 749 262 A1**
(43) Date of publication of application: **27.05.2026**
(21) Application number: 24843527.3
(22) Date of filing: 18.07.2024
(51) Int. Cl.: G01N 11/00, G01N 35/00, G01N 35/10

(54) **BLOOD VISCOSITY MEASUREMENT MODULE FOR AUTOMATIC BLOOD VISCOMETER**

(30) Priority: 19.07.2023 KR 20230093910; 08.07.2024 KR 20240089871
(71) Applicant: INDUSTRIAL COOPERATION FOUNDATION JEONBUK NATIONAL UNIVERSITY, Jeonju-si, Jeollabuk-do 54896 (KR)
(72) Inventor: LEE, Dong Hwan, Jeonju-si Jeonbuk-do 54900 (KR)
(74) Representative: Jung, Minkyu
(86) International application number: PCT/KR2024/010375
(87) International publication number: WO 2025/018813

(57) **Abstract**

Provided is a blood viscosity measurement module for an automatic blood viscosity measurement device, and more specifically, a blood viscosity measurement module for an automatic blood viscosity measurement device that can measure viscosity of a blood sample injected into a test kit with high accuracy through a constant temperature maintenance means, simultaneously process viscosity measurement for a plurality of blood samples through a multi-channel structure, and minimize measurement errors through a structure in which the test kit can be brought into close contact with a blood flow detection sensor.

## Description

### Technical Field

The present invention relates to a blood viscosity measurement module for an automatic blood viscosity measurement device, and more specifically, to a blood viscosity measurement module for an automatic blood viscosity measurement device that may measure viscosity of a blood sample injected into a test kit with high accuracy through a constant temperature maintenance means, simultaneously process viscosity measurement for a plurality of blood samples through a multi-channel structure, and minimize measurement errors through a structure in which the test kit may be brought into close contact with a blood flow detection sensor.

### Background Art

Blood viscosity is a physical property that represents the flow resistance due to the flow of blood in blood vessels and may be specifically divided into whole blood viscosity and plasma viscosity. An abnormal increase in blood viscosity causes an increase in shear stress and flow resistance acting on inner walls of blood vessels, which significantly increases the risk of developing acute cardiovascular disease and microvascular disease.

In addition, the plasma viscosity is not only used to diagnose inflammatory conditions in the body, but also is one of the main causes of increased whole blood viscosity.

The whole blood viscosity shows flow characteristics in which viscosity continuously changes according to the systole and diastole of the heart, and the reason is because the viscosity decreases when the blood flows at a high speed (when a shear rate is high) due to the mutual complex influence of red blood cells and plasma proteins in the whole blood, and conversely, the viscosity increases when the blood flows at a low speed (when the shear rate is low).

A fluid that shows the flow characteristics is called a non-Newtonian fluid, and in order to properly understand the non-Newtonian flow characteristics of blood, it is necessary to accurately measure the whole blood viscosity for the entire shear rate (for example, 1 to 1,000 s^-1).

Recent blood viscosity measurement devices allow the blood obtained from the body to pass through a flow restrictor tube, and measure the flow characteristics of blood within the flow restrictor tube to measure blood viscosity and blood cell aggregation rate.

Korean Utility Model No. 20-0331884 (Apparatus to measure simultaneously both blood viscosity and cell aggregation) which is a conventional technology is disclosed.

However, since a device operator has to manually inject blood through a syringe to measure blood viscosity, it is difficult to supply blood at a constant pressure and flow rate, and thus, there is a problem that it is difficult to measure blood viscosity under the same condition.

In addition, since it is done manually, there is a problem that the work time is lengthened, and since it is done manually, a blood-borne infection problem also occurs frequently.

In order to solve the problem, an automated blood viscosity measurement device is being developed, but there is a problem that the work time is lengthened because when the viscosity measurement for one blood sample is completed, the viscosity measurement for the next blood sample is performed.

In addition, when measuring a large number of blood samples, when the blood sample is a late-order blood sample, the viscosity measurement is performed in a state where red blood cells, or the like settle over time, resulting in a problem of reduced accuracy of the viscosity measurement.

Also, the conventional technology has a problem in that viscosity of multiple blood samples may not be simultaneously measured due to a single channel and has a structural limitation that may cause measurement errors when measuring viscosity.

### Disclosure of Invention

### Technical Problem

The present invention is to solve the problem of the conventional technology described above, and an object of the present invention is to provide a blood viscosity measurement module for an automatic blood viscosity measurement device which may simultaneously process multiple blood samples with high accuracy and minimize measurement errors.

### Solution to Problem

In order to achieve the above object, a blood viscosity measurement module for an automatic blood viscosity measurement device according to the present invention includes a viscosity measurement unit configured to measure viscosity of a blood sample injected into a test kit gripped by a third gripper and transferred by a transfer actuator; and a controller configured to check a state of the viscosity measurement unit and control an operation of the viscosity measurement unit, wherein the viscosity measurement unit includes a channel module into which the test kit gripped and transferred by the third gripper is inserted in a vertical direction and in which the test kit is mounted; a temperature maintenance means for heating or cooling the test kit mounted on the channel module such that the test kit maintains a predetermined temperature; and a viscosity measurement means for measuring the viscosity of the blood sample injected into the test kit.

Here, the channel module may be configured with a plurality of channel modules to have a multi-channel structure, and the controller may control the transfer actuator such that test kits gripped and transferred by the third gripper are sequentially mounted on empty channel modules among the plurality of channel modules.

Here, the temperature maintenance means may include a temperature sensor configured to detect temperature of the test kit mounted on the channel module; a heater configured to heat the test kit mounted on the channel module; a cooling fan configured to cool the test kit mounted on the channel module; and a temperature controller configured to selectively operate one of the heater and the cooling fan such that temperature detected by the temperature sensor may maintain a set temperature.

Here, the test kit may include a U-shaped tube through which, when a blood sample is injected into an upper portion on one side, the injected blood sample flows in another direction due to a height difference, and the viscosity measurement means may include a blood flow detection sensor provided on one surface of the channel module and configured to detect a speed of the blood sample flowing to another side of the U-shaped tube; and a viscosity calculator configured to calculate viscosity of the blood sample using the speed of the blood sample detected by the blood flow detection sensor.

Here, the channel module may include an elastic spring configured to elastically pressurize an inserted test kit in a direction in which the test kit comes into contact with one surface on which the blood flow detection sensor is provided.

In addition, in the blood viscosity measurement module for an automatic blood viscosity measurement device according to the present invention, the viscosity measurement unit may include a kit detection sensor configured to detect whether the test kit is mounted on the channel module; and a progress notification lamp configured to visually display information detected by the kit detection sensor and progress of measurement in the channel module.

In addition, in the blood viscosity measurement module for an automatic blood viscosity measurement device according to the present invention, the viscosity measurement unit may further include a vibration-proof means disposed at a lower portion of the channel module to attenuate vibration transmitted to the channel module.

### Advantageous Effects of Invention

With the above configuration, a blood viscosity measurement module for an automatic blood viscosity measurement device according to the present invention has an advantage of measuring viscosity of a blood sample injected into a test kit with high accuracy through a constant temperature maintenance means, simultaneously processing viscosity measurement for a plurality of blood samples through a multi-channel structure, and minimizing measurement errors through a structure in which the test kit may be brought into close contact with a blood flow detection sensor.

### Brief Description of Drawings

FIGS. 1 and 2 are perspective views of an automatic blood viscosity measurement device according to an embodiment of the present invention.
FIG. 3 is a plan view of the automatic blood viscosity measurement device according to the embodiment of the present invention.
FIG. 4 is a perspective view of a housing according to an embodiment of the present invention.
FIGS. 5 and 6 are perspective views of a first input portion according to an embodiment of the present invention.
FIGS. 7 and 8 are perspective views of a second input portion according to an embodiment of the present invention.
FIGS. 9 and 10 are perspective views of a third input portion according to an embodiment of the present invention.
FIG. 11 is a perspective view of a transfer unit according to an embodiment of the present invention.
FIGS. 12 to 14 are use state views of first, second, and third transfer units according to an embodiment of the present invention.
FIG. 15 is a front perspective view of a main unit according to an embodiment of the present invention.
FIGS. 16 to 18 are perspective views of a preprocessing unit according to an embodiment of the present invention.
FIG. 19 is a use state view of a blood suction/mixing unit according to an embodiment of the present invention.
FIGS. 20 to 23 are perspective views of a viscosity measurement unit according to an embodiment of the present invention.

### Best Mode for Carrying out the Invention

a blood viscosity measurement module for an automatic blood viscosity measurement device according to the present invention may include a viscosity measurement unit configured to measure viscosity of a blood sample injected into a test kit gripped by a third gripper and transferred by a transfer actuator; and a controller configured to check a state of the viscosity measurement unit and control an operation of the viscosity measurement unit, wherein the viscosity measurement unit includes a channel module into which the test kit gripped and transferred by the third gripper is inserted in a vertical direction and in which the test kit is mounted; a temperature maintenance means for heating or cooling the test kit mounted on the channel module such that the test kit maintains a predetermined temperature; and a viscosity measurement means for measuring the viscosity of the blood sample injected into the test kit.

### Mode for the Invention

Hereinafter, an automatic blood viscosity measurement device according to the present invention will be described in detail with reference to embodiments illustrated in the drawings.

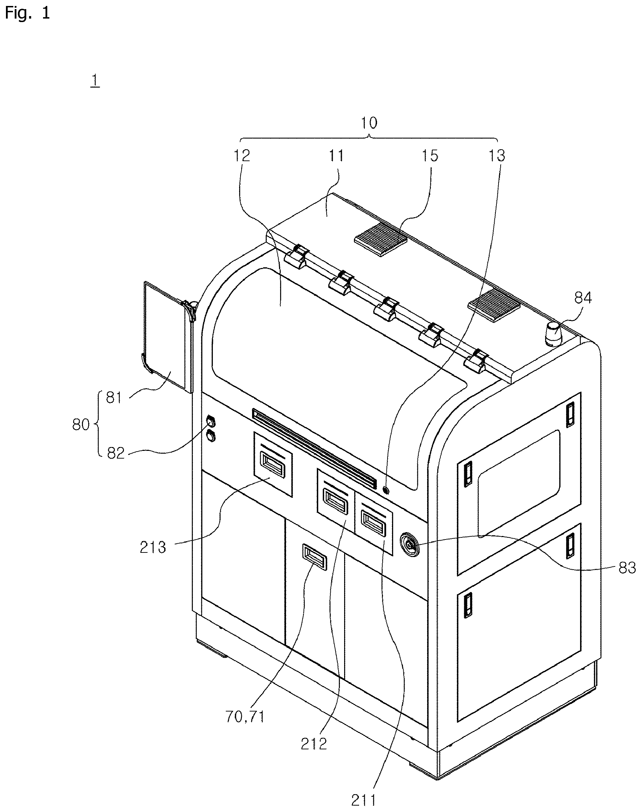

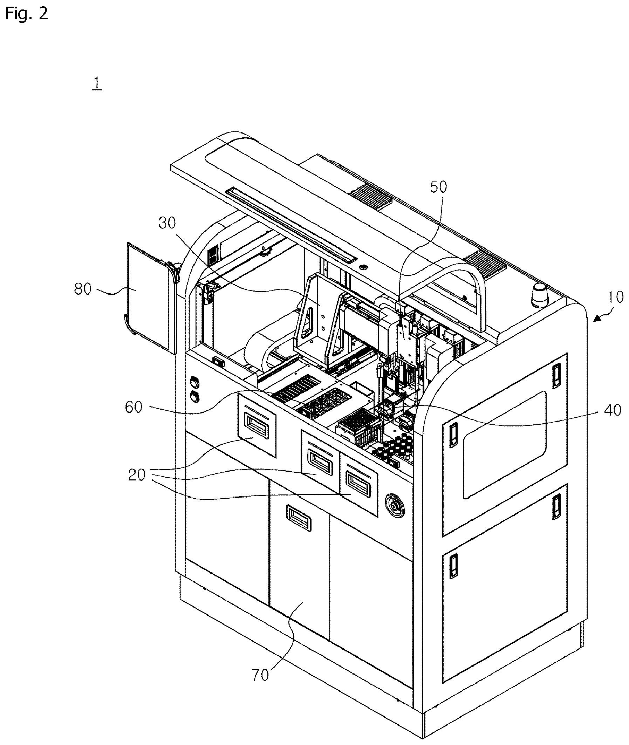

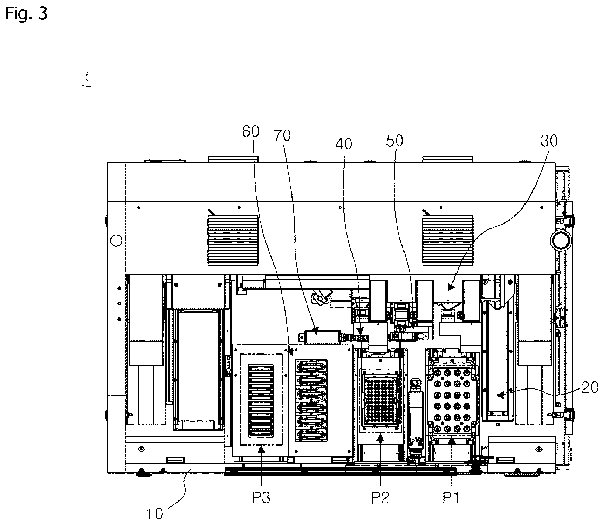

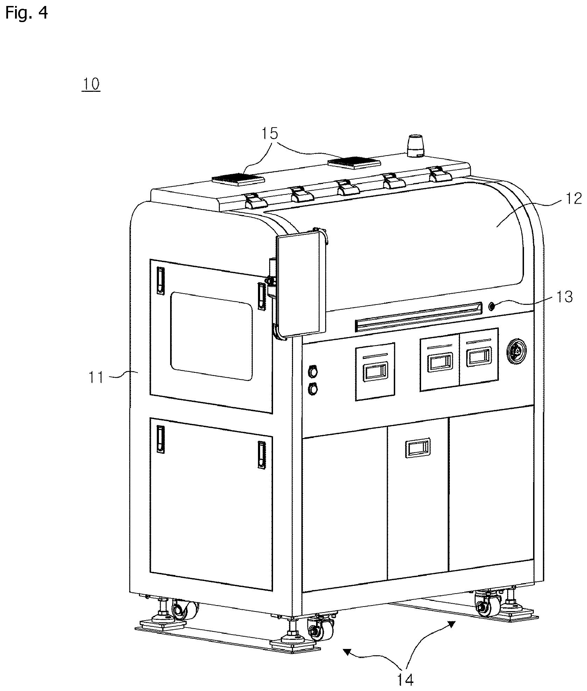

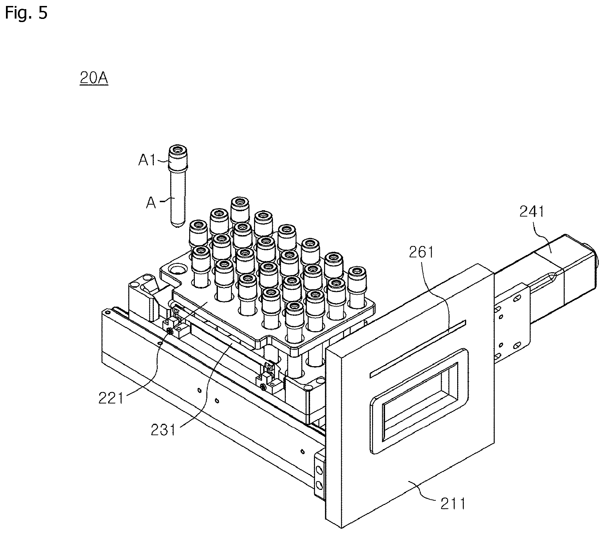

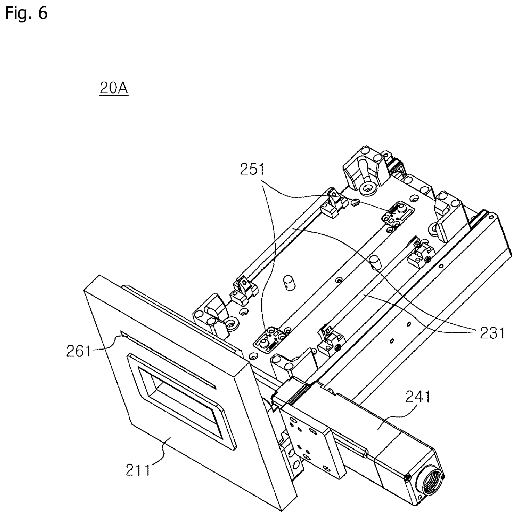

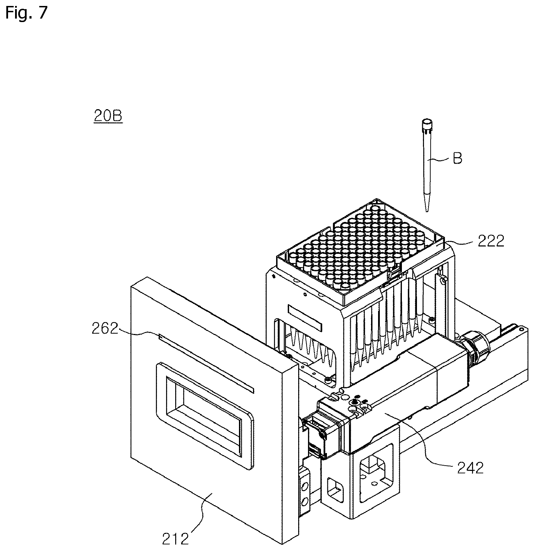

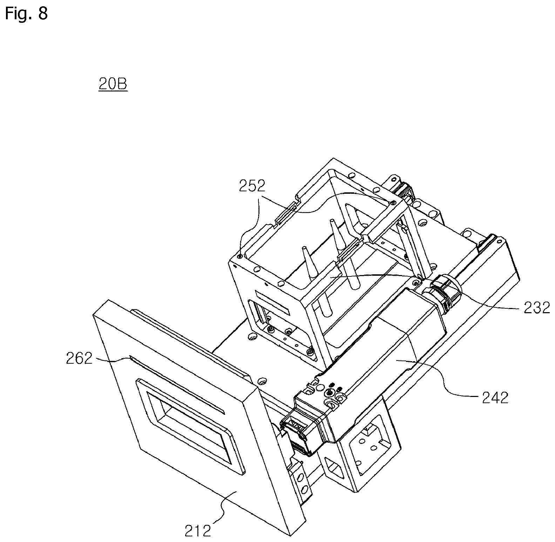

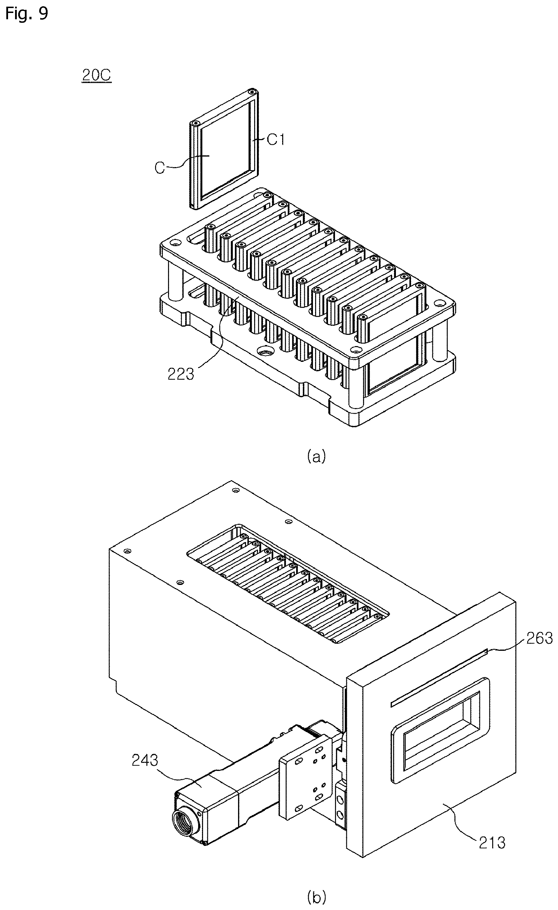

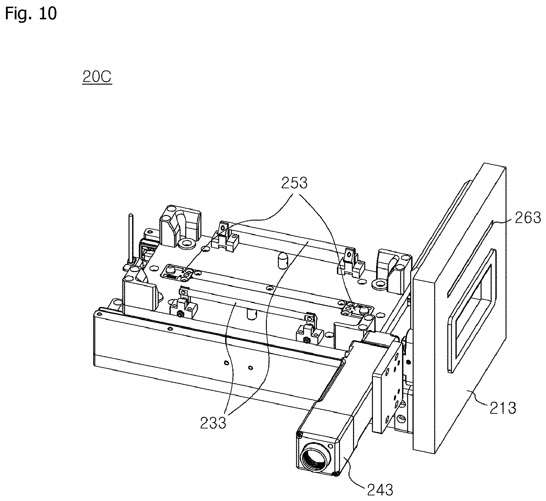

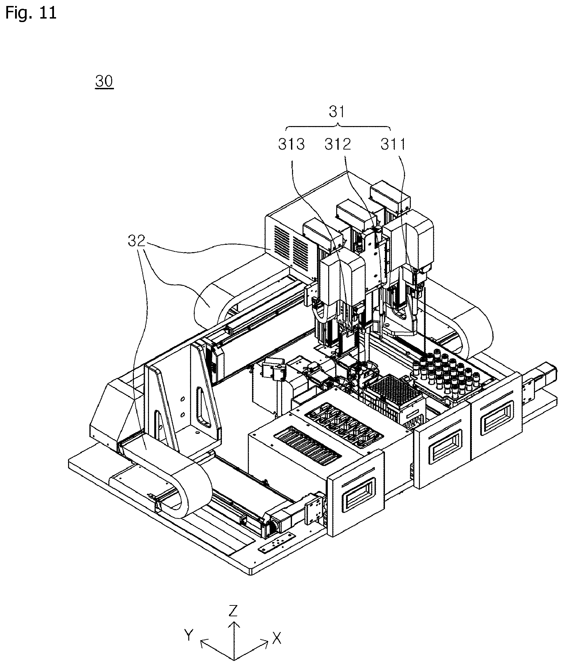

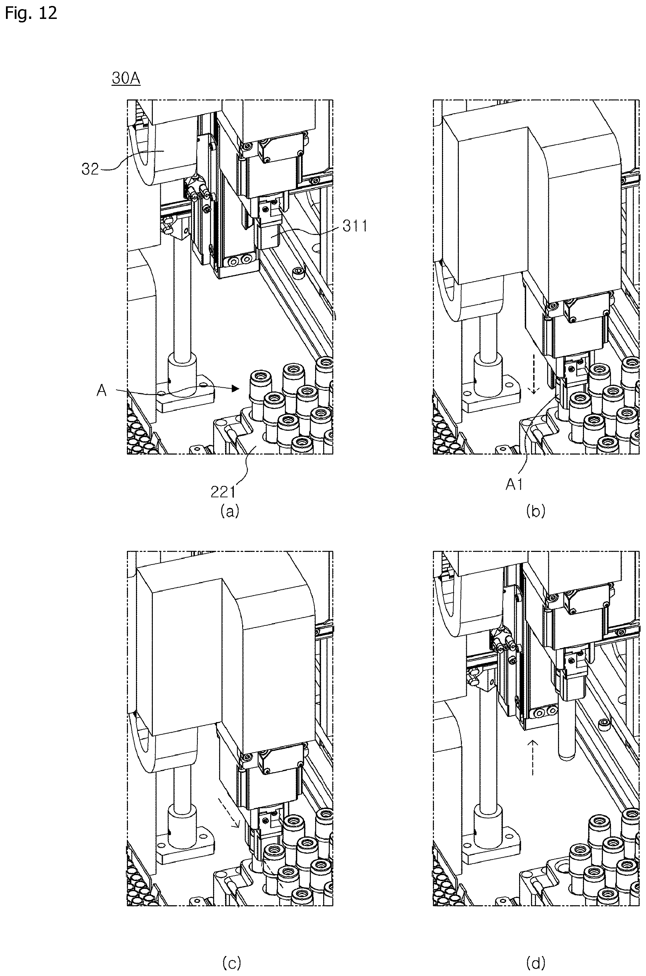

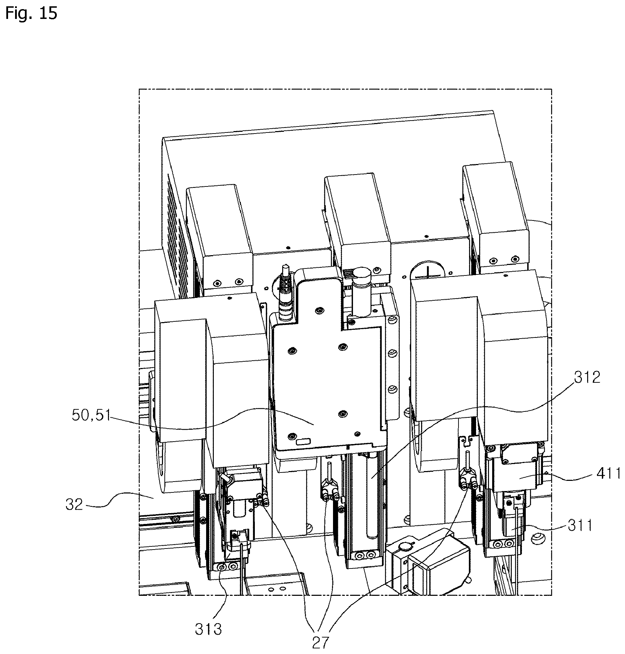

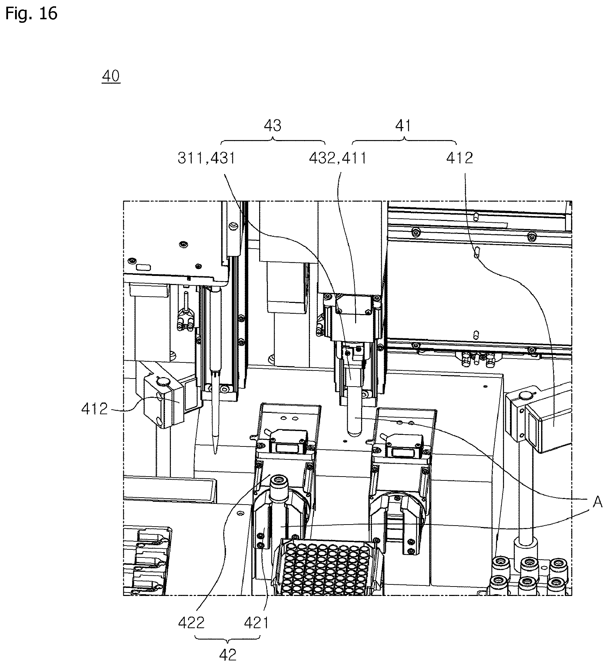

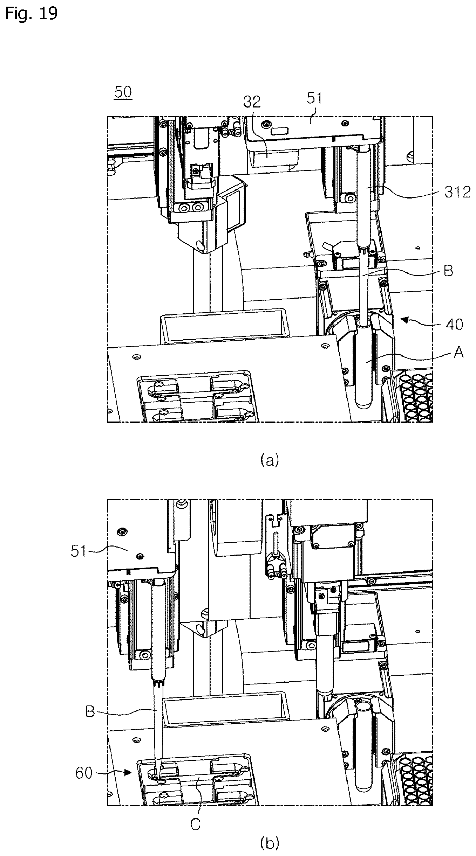

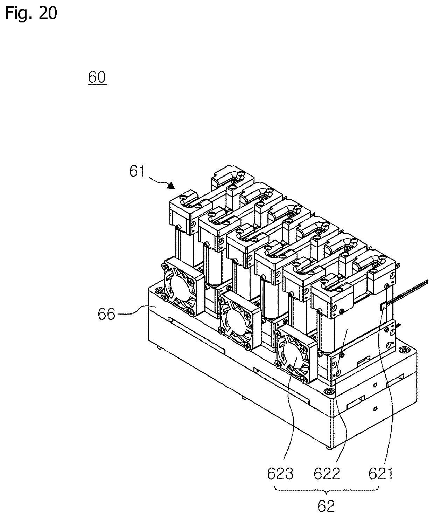

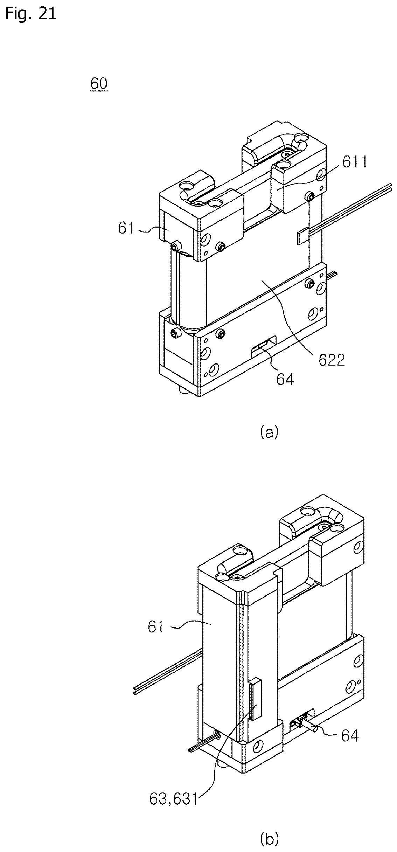

FIGS. 1 and 2 are perspective views of an automatic blood viscosity measurement device according to an embodiment of the present invention, FIG. 3 is a plan view of the automatic blood viscosity measurement device according to the embodiment of the present invention, FIG. 4 is a perspective view of a housing according to an embodiment of the present invention, FIGS. 5 and 6 are perspective views of a first input portion according to an embodiment of the present invention, FIGS. 7 and 8 are perspective views of a second input portion according to an embodiment of the present invention, FIGS. 9 and 10 are perspective views of a third input portion according to an embodiment of the present invention, FIG. 11 is a perspective view of a transfer unit according to an embodiment of the present invention, FIGS. 12 to 14 are use state views of first, second, and third transfer units according to an embodiment of the present invention, FIG. 15 is a front perspective view of a main unit according to an embodiment of the present invention, FIGS. 16 to 18 are perspective views of a preprocessing unit according to an embodiment of the present invention, FIG. 19 is a use state view of a blood suction/mixing unit according to an embodiment of the present invention, and FIGS. 20 to 23 are perspective views of a viscosity measurement unit according to an embodiment of the present invention.

Referring to FIGS. 1 to 3, an automatic blood viscosity measurement device 1 according to an embodiment of the present invention includes a housing 10, an input portion 20, a transfer unit 30, a preprocessing unit 40, a blood suction/injection unit 50, a viscosity measurement unit 60, a waste disposal unit 70, and a controller 80.

Here, the blood viscosity measurement module for an automatic blood viscosity measurement device, according to the present invention, may be configured by a combination of the viscosity measurement unit 60 and the controller 80.

The housing 10 may form the entire appearance of the automatic blood viscosity measurement device 1 according to an embodiment of the present invention, and include a housing body 11, a management door 12, a door locking means 13, a lower support 14, and a blower fan 15.

The housing body 11 may provide an inner space in which other components of the automatic blood viscosity measurement device 1 according to an embodiment of the present invention may be built, and may be configured by a combination of a frame and a cover plate.

The management door 12 may be made of a see-through material, such as transparent acrylic, such that a user may observe an inner state of the housing 10, and when management of the device 1 is required, the management door 12 may cover an upper front surface of the housing body 11 and may be installed to be openable such that the inside of the housing 10 may be opened.

The door locking means 13 may be configured to lock the management door 12 without being opened, and may be composed of an interlock, a solenoid, a manual locking key, and so on.

Here, the controller 80 is configured to control the door locking means 13 such that the management door 12 may be locked or opened according to a user's operation or a confirmed state of the device 1.

That is, the management door 12 is controlled to basically maintain a locked state by the door locking means 13 for safety when the device 1 is in an operating state, and is controlled to be openable when the device 1 is stopped by an operation of the power button 82, an emergency stop switch 83, or so on.

The lower support 14 is configured to support a lower portion of the housing body 11 as illustrated in FIG. 4, and may facilitate movement of the device 1 through moving wheels respectively provided at lower corners of the housing body 11 and may adjust a level of the device 1 and fix a position of the device 1 through an adjustable seat that may adjust a height and suck vibration when a installation position of the device 1 is determined, and may minimize the vibration generated by an operation of the device 1.

The blower fan 15 may be configured to discharge the heat generated inside the housing 10 to the outside and be installed in plural units in an upper portion of the housing body 11.

Meanwhile, to increase cooling efficiency of the blower fan 15, the housing 10 may be provided with an air sucking hole (not illustrated) for sucking external air into the inside.

The input portion 20 is configured to insert a blood collection tube A, a pipette tip B, and a test kit C respectively into first, second, and third positions P1, P2, and P3 inside the housing 10, and in an embodiment of the present invention, the input portion 20 is configured with first, second, and third input portions 20A, 20B, and 20C.

Embodiments of the first, second, and third positions P1, P2, and P3 inside the housing 10 are illustrated in a plan view of FIG. 3.

As illustrated in FIGS. 5 and 6 , the first input portion 20A is configured to insert the blood collection tube A containing a blood sample and having a sealing cap A1 fastened to an upper portion into a first position P1 inside the housing 10, and includes a first replacement drawer 211, a first tray 221, a first tray holder 231, a first drawer locking means 241, a first tray detection sensor 251, and a first state indicator lamp 261.

The first replacement drawer 211 is configured to be supported on the housing 10 so as to be slidingly movable back and forth between the outside of the housing 10 and the first position P1 such that the blood collection tube A may be replaced from the outside of the housing 10.

The first tray 221 is configured such that a plurality of blood collection tubes A are vertically mounted in the first tray 221 in an aligned state.

In an embodiment of the present invention, the first tray 221 is configured such that 24 blood collection tubes A may be vertically mounted on the first tray 221 in a 4x6 arrangement.

The first tray holder 231 is configured to fix the first tray 221 in a state where the first tray 221 is safely placed in the first replacement drawer 211.

The first tray holder 231 plays a role of preventing the first tray 221 from shaking when the first gripper 311 of the transfer unit 30 grips and lifts the blood collection tube A vertically mounted on the first tray 221.

The first drawer locking means 241 is configured to lock the first replacement drawer 211 in a state where the first replacement drawer 211 is located at the first position P1.

The first drawer locking means 241 may be controlled by the controller 80 and be configured with an interlock, a solenoid, a manual lock key, and so on to prevent the first replacement drawer 211 from being unexpectedly opened in a state where the device 1 is in operation.

The first tray detection sensor 251 is configured to detect whether the first tray 221 is safely placed in a correct position of the first replacement drawer 211.

The first tray detection sensor 251 may be configured to detect at least two points, preferably three points, so as to detect whether the first tray 221 is horizontally mounted in the correct position of the first replacement drawer 211.

The first state display lamp 261 is provided near the first replacement drawer 211 and is configured to visually display, through a color change, a state of the first input portion 20A related to the blood collection tube A among states of the device 1 determined by the controller 80.

That is, the first state display lamp 261 is provided on the front of the first replacement drawer 211 and may be configured to visually display a shortage state of the blood collection tube A, a poor placement state of the first tray 221, a poor locking state of the first replacement drawer 211, and so on, in different colors, such as red, blue, green, orange, white, and so on.

As illustrated in FIGS. 7 and 8, the second input portion 20B is configured to input the pipette tip B that is disposable and may suck and dispense a blood sample into a second position P2 inside the housing 10, and includes a second replacement drawer 212, a second tray 222, a second tray holder 232, a second drawer locking means 242, a second tray detection sensor 252, and a second state indicator lamp 262.

The second replacement drawer 212 is configured to be supported on the housing 10 so as to be slidingly movable back and forth between the outside of the housing 10 and the second position P2 such that the pipette tip B may be replaced from the outside of the housing 10.

The second tray 222 is configured such that a plurality of pipette tips B are vertically mounted in the second tray 222 in an aligned state.

In an embodiment of the present invention, the second tray 222 is configured to enable 96 pipette tips B to be vertically mounted in an 8×12 arrangement.

The second tray 222 may be manufactured with a dual structure of an upper holder and a lower holder.

The second tray holder 232 is configured to fix the second tray 222 in a state where the second tray 222 is safely placed respectively in the second replacement drawer 212.

The second tray holder 232 prevents the second tray 222 from shaking when a second gripper 312 of the transfer unit 30 grips and lifts the pipette tip B vertically mounted on the second tray 222.

The second drawer locking means 242 is configured to lock the second replacement drawer 212 in a state where the second replacement drawer 212 is located at the second position P2.

The second drawer locking means 242 is controlled by the controller 80 and may be configured to prevent the second replacement drawer 212 from being unexpectedly opened in a state where the device 1 is in operation, and may be configured with an interlock, a solenoid, a manual lock key, and so on.

The second tray detection sensor 252 is configured to detect whether the second tray 222 is safely placed in a correct position of the second replacement drawer 212.

The second tray detection sensor 252 may be configured to detect at least two points, preferably three points, so as to detect whether the second tray 222 is horizontally mounted in the correct position of the second replacement drawer 212.

The second state display lamp 262 is provided near the second replacement drawer 211 and is configured to visually display, through a color change, a state of the second input portion 20B related to the pipette tip B among states of the device 1 determined by the controller 80.

That is, the second state indicator lamp 262 may be provided on the front of the second replacement drawer 212 and may be configured to visually display a shortage state of the pipette tip B, a poor placement state of the second tray 222, a poor locking state of the second replacement drawer 212, and so on, in different colors, such as red, blue, green, orange, white, and so on, or any combination thereof.

As illustrated in FIGS. 9 and 10, the third input portion 20C is configured to input the test kit C, which is disposable and used for blood viscosity measurement, into the third position P3 inside the housing 10, and includes a third replacement drawer 213, a third tray 223, a third tray holder 233, a third drawer locking means 243, a third tray detection sensor 253, and a third state indicator lamp 263.

The third replacement drawer 213 is configured to be supported on the housing 10 so as to be slidingly movable back and forth between the outside of the housing 10 and the third position P3 such that the test kit C may be replaced from the outside of the housing 10.

The third tray 223 is configured such that a plurality of test kits C are vertically mounted in the third tray 223 in an aligned state.

In an embodiment of the present invention, the third tray 223 is configured such that 12 test kits C are vertically mounted in the third tray 223 in a single row.

Meanwhile, the number of blood collection tubes A, pipette tips B, and test kits C that may be mounted respectively on the first, second, and third trays 221, 222, and 223 is a multiple of each other.

With the above configuration, the automatic blood viscosity measurement device according to the present invention may minimize the time required for replacement by interlocking replacement cycles of the blood collection tube A, the pipette tip B, and the test kit C.

To this end, as described above, in an embodiment of the present invention, 24 blood collection tubes A may be mounted in the first tray 221, 96 pipette tips B may be mounted in the second tray 222, and 12 test kits C may be mounted in the third tray 223.

The third tray holder 233 is configured to fix the third tray 223 in a state where the third tray 223 is safely placed in the third replacement drawer 213.

The third tray holder 233 prevents the third tray 223 from shaking when a third gripper 313 of the transfer unit 30 grips and lifts the test kit C vertically mounted in the third tray 223.

The third drawer locking means 243 is configured to lock the third replacement drawer 213 in a state where the third replacement drawer 213 is located at the third position P3.

The third drawer locking means 243 is controlled by the controller 80 and may be configured to prevent the third replacement drawer 213 from being opened unexpectedly in a state where the device 1 is in operation, and may be configured with an interlock, a solenoid, a manual lock key, and so on.

The third tray detection sensor 253 is configured to detect whether the third tray 223 is safely placed in a correct position of the third replacement drawer 213.

The third tray detection sensor 253 may be configured to detect at least two points, preferably three points, so as to detect whether the third tray 223 is horizontally mounted in the correct position of the third replacement drawer 213.

The third state display lamp 263 is provided near the third replacement drawer 211 and is configured to visually display, through a color change, a state of the third input portion 20C related to the test kit C among states of the device 1 determined by the controller 80.

That is, the third state indicator lamp 263 may be provided on the front of the third replacement drawer 213 and may be configured to visually display a shortage state of the test kit C, a poor placement state of the third tray 223, a poor locking state of the third replacement drawer 213, and so on, in different colors, such as red, blue, green, orange, white, and so on.

Meanwhile, the input portion 20 includes an input material detection sensor 27 that detects positions and quantities of the blood collection tube A, the pipette tip B, and test kit C respectively input to the first, second, and third positions P1, P2, and P3.

In an embodiment of the present invention, the input material detection sensor 27 is attached to and moved by a transfer actuator 32 of a transfer unit 30 as illustrated in FIG. 12, and may be configured with a beam-type scanning sensor having a built-in amplifier capable of detecting the positions and quantities of the blood collection tube A, the pipette tip B, and the test kit C that are respectively input to the first, second, and third positions P1, P2, and P3.

Meanwhile, the input material detection sensor 27 may be configured to apply a laser or visible light beam thereto such that a user may directly and visually check a position of a detection spot to enable immediate detection of measurement errors.

The input material detection sensor 27 may be configured to detect the positions and quantities of the blood collection tube A, the pipette tip B, and the test kit C input to the first, second, and third positions P1, P2, and P3 at an initial operation of the device 1, and a subsequent state may be determined by the controller 80, and the controller 80 may determine a state of the device 1 according to the progress of measurement based on the information detected by the input material detection sensor 27 through software.

The transfer unit 30 is provided with grippers 31 and transfer actuators 32 configured to respectively transfer the blood collection tube A, the pipette tip B, and the test kit C, as illustrated in FIG. 11.

The grippers 31 are configured with first, second, and third grippers 311, 312, and 313 capable of respectively gripping the blood collection tube A, the pipette tip B, and the test kit C input to the housing 10 by the input unit 20.

In an embodiment of the present invention, the first gripper 311 is configured in the form of forceps capable of gripping the sealing cap A1 of the blood collection tube A from above.

FIG. 12 illustrates a first transfer unit 30A that transfers the blood collection tube A, and illustrates a state in which the first gripper 311 grips one of a plurality of blood collection tubes A vertically mounted in the first tray 221 and then is transferred by the transfer actuator 32.

That is, the first gripper 311 in the form of forceps may move to an upper portion of the blood collection tube A of a gripping target as illustrated in (a) of FIG. 12, vertically move downward from that position as illustrated in (b) of FIG. 12, grip the sealing cap A1 of the blood collection tube A as illustrated in (c) of FIG. 12, and vertically move upward as illustrated in (d) of FIG. 12, and accordingly, the gripped blood collection tube A may be moved upward.

Thereafter, the blood collection tube A gripped by the first gripper 311 is moved in the X-axis direction, Y-axis direction, or Z-axis direction by an operation of the transfer actuator 32 under the control of the controller 80 and is transferred to a predetermined position, such as a scan position or a mixing position.

In addition, in an embodiment of the present invention, the second gripper 312 is formed in a shape in which a rear end of the pipette tip B may be forcibly inserted into the second gripper 312 and fixed thereon.

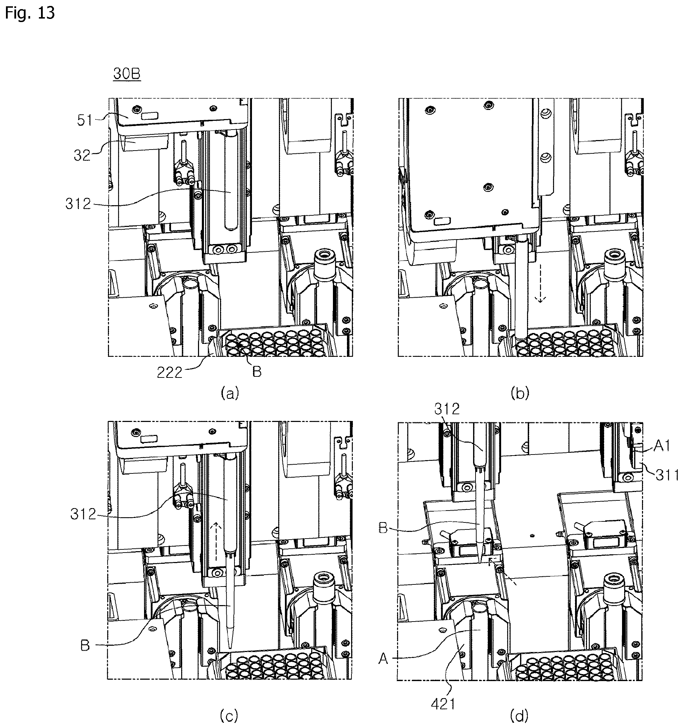

FIG. 13 illustrates a second transfer unit 30B that transfers the pipette tip B, and illustrates a state in which the second gripper 312 grips one of a plurality of pipette tips B vertically mounted in the second tray 222 and then is transferred by the transfer actuator 32.

That is, the second gripper 312 is moved to an upper portion of the pipette tip B of a gripping target as illustrated in (a) of FIG. 13, vertically moved downward from that position to forcibly insert a rear end of the pipette tip B into the second gripper 312 and fix thereon as illustrated in (b) of FIG. 12, and vertically moved upward to transfer the forcibly inserted and fixed pipette tip B upward as illustrated in (c) of FIG. 12.

Thereafter, as illustrated in (d) of FIG. 13, the pipette tip B fixed by the second gripper 312 is moved in the X-axis direction, Y-axis direction, or Z-axis direction by an operation of the transfer actuator 32 under the control of the controller 80, transferred to an upper portion of the blood collection tube A having a body portion held by blood collection tube forceps 421 in a state where the sealing cap A1 is separated so as to prepare for blood suction, and when the blood suction is completed, the pipette tip B is moved to an upper portion of the test kit C mounted in the viscosity measurement unit 70 and prepares for blood dispensing.

In addition, in an embodiment of the present invention, the third gripper 313 may be configured in the form of forceps capable of gripping an upper central portion of the test kit C from above.

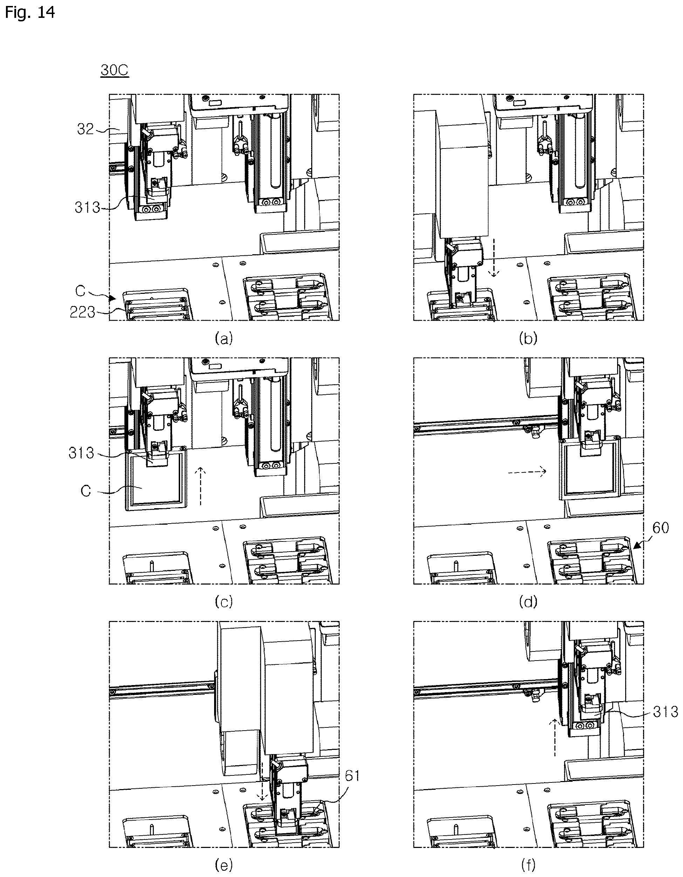

FIG. 14 illustrates a third transfer unit 30C that transfers the test kit C, and illustrates a state in which the third gripper 313 grips one of a plurality of test kits C vertically mounted in the third tray 223 and then, is transferred by the transfer actuator 32.

That is, the third gripper 313 is moved to an upper portion of the test kit C of a gripping target as illustrated in (a) of FIG. 14, vertically moved downward from that position and then the third gripper 313 in the form of forceps grips an upper central portion of the test kit C as illustrated in (b) of FIG. 14, and vertically moved upward to enable the gripped test kit C to be transferred upward as illustrated in (c) of FIG. 14.

Thereafter, the test kit C gripped by the third gripper 313 is moved in the X-axis direction, Y-axis direction, or Z-axis direction by an operation of the transfer actuator 32 under the control of the controller 80 and moved to an upper portion of the viscosity measurement unit 60 as illustrated in (d) of FIG. 14, vertically moved downward from that position and enables the gripped test kit C to be inserted and mounted in the channel unit 61 as illustrated in (e) of FIG. 14, and vertically moved upward in a state where gripping of the test kit C is released as illustrated in (f) of FIG. 14.

Meanwhile, in the first and third grippers 311 configured in the form of forceps, a groove for preventing slipping may be formed on a contact surface of the blood collection tube A or test kit C, and a gripping force monitoring technology may be applied to prevent damage to the blood collection tube A or test kit C due to excessive gripping force.

The transfer actuator 32 is configured to move the first, second, and third grippers 311, 312, and 313 in an interlocking manner.

In an embodiment of the present invention, as illustrated in FIG. 11, the transfer actuator 32 may be configured as a linear actuator having a structure in which the first, second, and third grippers 311, 312, and 313 are arranged in parallel on the X axis, the first, second, and third grippers 311, 312, and 313 may integrally move forward, backward, left, and right along the X axis and the Y axis, and the first, second, and third grippers 311, 312, and 313 may independently move up and down along the Z axis.

Through a combination of the first, second, and third grippers 311, 312, and 313 and the transfer actuator 32 and a transfer sequence control by the controller 80, the device 1 according to the present invention may automate the entire process, reduce manufacturing costs, and be made to have a medium size.

The preprocessing unit 40 is configured to preprocess the blood collection tube A gripped and transferred by the first gripper 311, and includes a scan means 41, a blood mixing means 42, and a cap separation means 43, as illustrated in FIGS. 16 to 18.

The scan means 41 is configured to obtain information of the blood collection tube A, and includes a first rotation motor 411 and a blood collection tube scanner 412 in an embodiment of the present invention.

The first rotation motor 411 is configured to rotate the first gripper 311 such that the blood collection tube A gripped by the first gripper 311 and transferred to a predetermined scan position rotates about the Z axis set as the center of rotation.

The blood collection tube scanner 412 is disposed at a scan position and configured to scan the blood collection tube A rotated by an operation of the first rotation motor 411 to obtain blood collection tube information indicated on the blood collection tube A.

That is, a barcode including the blood collection tube information is typically attached to the blood collection tube A, and since the attached barcode is not always aligned in a direction facing the blood collection tube scanner 412, the blood collection tube A is rotated horizontally by the first rotation motor 411 in a state where the blood collection tube A is moved to a scan position by the transfer unit 30, and accordingly, the barcode attached to the blood collection tube A may be scanned by the blood collection tube scanner 412 during a process of rotating the blood collection tube A.

In addition, it is preferable to use equipment having a scan range greater than a certain level for the blood collection tube scanner 412, so as to resolve a scan error due to tilt or an attachment position of the barcode attached to the blood collection tube A, and in some cases, the scan range may be expanded by applying an actuator (not illustrated) that may move the blood collection tube scanner 412 in the Z-axis direction.

The blood mixing means 42 is configured to mix a blood sample contained in the blood collection tube A, and includes blood collection tube forceps 421 and a mixing rotation motor 422 in an embodiment of the present invention.

The blood collection tube forceps 421 are configured to hold a body portion of the blood collection tube A gripped by the first gripper 311 and transferred to a predetermined mixing position.

The mixing rotation motor 422 is configured to rotate the blood collection tube forceps 421.

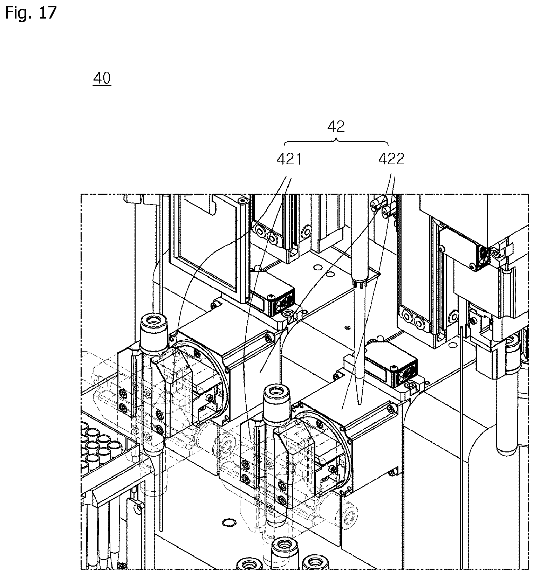

As illustrated in FIG. 17, the blood collection tube A held by the blood collection tube forceps 421 is rotated 360° around the X axis or Y axis by the mixing rotation motor 422, and accordingly, it is possible to completely prevent an erythrocyte sedimentation phenomenon that may occur during the waiting time before the blood sample contained therein is mixed and sucked by the blood suction/injection unit 50, and to maintain a uniform blood composition.

The cap separation means 43 is configured to separate the sealing cap A1 from the blood collection tube A, and includes cap forceps 431 and a cap rotation motor/actuator 432 in an embodiment of the present invention.

The cap forceps 431 are configured to hold the sealing cap A1 fastened to an upper end of the blood collection tube A in a state where the body portion of the blood collection tube A is held by the blood collection tube forceps 421.

In an embodiment of the present invention, the cap forceps 431 may be replaced with the first gripper 311, as illustrated in FIG. 13.

The cap rotation motor/actuator 432 is configured to rotate the cap forceps 431 about the Z axis set as the center of rotation and simultaneously move the cap forceps 431 up and down.

In an embodiment of the present invention, the cap rotation motor/actuator 432 may be replaced with a combination of the first rotation motor 411 and the transfer actuator 32, as illustrated in FIG. 13.

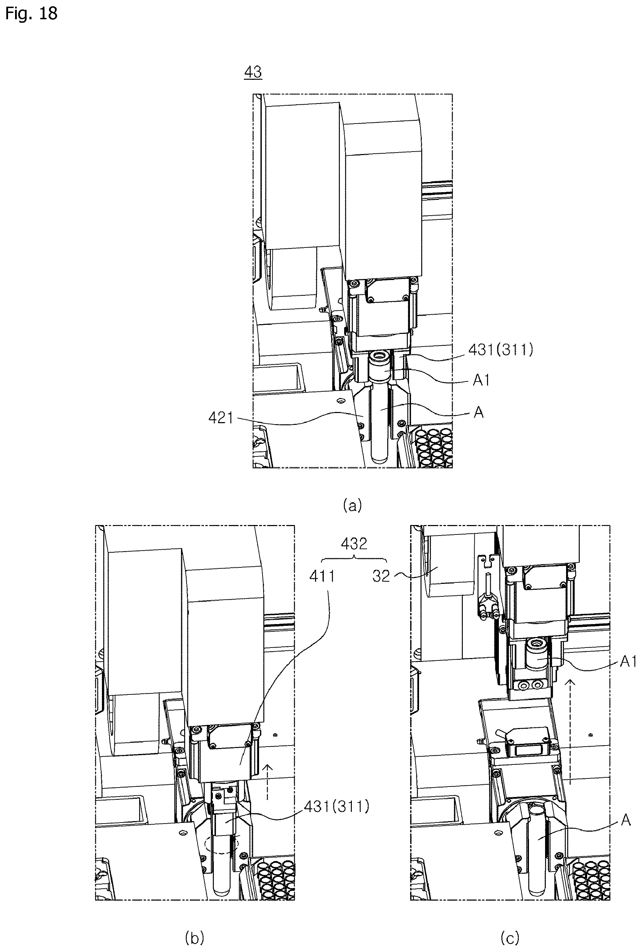

In a state where the body portion of the blood collection tube A is held by the blood collection tube forceps 421 as illustrated in (a) of FIG. 18, the sealing cap A1 is rotated and moved upward by a combination of the cap forceps 431 and the cap rotation motor/actuator 432 as illustrated in (b) of FIG. 18, and is separated from the blood collection tube A as illustrated in (c) of FIG. 18.

The blood collection tube A in which the sealing cap A1 is separated by the cap separation means 43 has a blood sample contained therein and sucked through the blood suction/injection unit 50, and when the blood sample is completely sucked from the blood collection tube A, the sealing cap A1 separated from the used blood collection tube A is reattached to the blood collection tube A by controlling the cap separation means 43 to operate in reverse by the controller 80, and the used blood collection tube A with the reattached sealing cap A1 is returned to the first position P1 under the control of the first gripper 311 and the transfer actuator 32.

Meanwhile, as illustrated in FIG. 16, the preprocessing unit 40 has a multi-space structure that may accommodate at least two blood collection tubes A and independently preprocess the accommodated blood collection tubes A, and herein, the controller 80 is configured to control the transfer actuator 32 such that the blood collection tubes A gripped and transferred by the first gripper 311 are sequentially accommodated in empty spaces among the multi-spaces of the preprocessing unit 40.

As described above, since the preprocessing unit 40 has a multi-space structure, the device 1 according to the present invention may control a transfer sequence by considering the relatively long blood mixing time and so on, and may reduce the processing time of the device 1.

The blood suction/injection unit 50 is configured to suck a blood sample from the blood collection tube A with the separated sealing cap A1 into the preprocessing unit 40 using the pipette tip B gripped and transferred by the second gripper 312 as illustrated in (a) of FIG. 19, and to inject the blood sample into the test kit C mounted in the viscosity measurement unit 60 as illustrated in (b) of FIG. 19.

In an embodiment of the present invention, the blood suction/injection unit 50 includes a piston 51 connected to a rear end of the pipette tip B through the second gripper 312 formed in a shape that allows the rear end of the pipette tip B to be forcibly inserted and fixed such that the pipette tip B may suck and dispense a blood sample.

The blood suction/injection unit 50 may apply a precision volume control technology within ±1.0% according to a change in electrical conductivity by using a disposable pipette tip B of a conductive type to precisely control the amount of blood sucked and discharged required for the test, or may also apply a precision volume control technology according to a change in pressure by using a disposable pipette tip B of a pressure type.

Meanwhile, when a blood sample is sucked from the blood collection tube A with the separated sealing cap A1 to the preprocessing unit 40 by using the pipette tip B, in relation to the blood suction/injection unit 50, the controller 80 controls the transfer actuator 32 such that the pipette tip B is lifted and lowered according to a height change of a blood sample contained in the blood collection tube A to enable suction at a certain depth based on a surface of the blood sample.

That is, the device 1 according to the present invention performs secondary mixing by the blood suction/injection unit 50 following the primary mixing by the blood mixing means 42 to mix a blood sample, and accordingly, accuracy of blood viscosity measurement through uniform mixing of the blood sample may be improved.

The test kit C is gripped and transferred by the third gripper 313 is mounted in the viscosity measurement unit 60, and the viscosity measurement unit 60 is configured to measure viscosity of a blood sample injected into the test kit C by the blood suction/injection unit 50.

The viscosity measurement unit 60 according to an embodiment of the present invention includes a channel module 61, a temperature maintenance means 62, a viscosity measurement means 63, a kit detection sensor 64, a progress notification lamp 65, and a vibration-proof means 66, as illustrated in FIGS. 20 to 23 .

The channel module 61 is configured to enable the test kit C gripped and transferred by the third gripper 313 to be inserted into the channel module 61 in a vertical direction and mounted thereon.

In an embodiment of the present invention, the channel module 61 has a gripper interference prevention groove 611 in the center of an upper portion to prevent interference with the third gripper 313, and includes an elastic spring 612 therein.

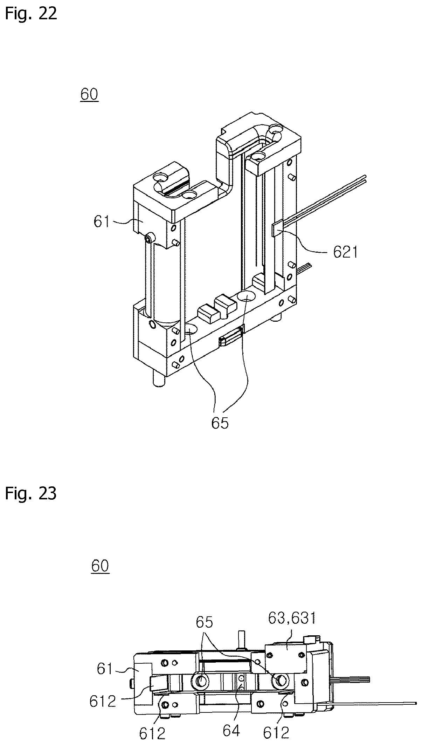

As illustrated in FIG. 23, the elastic spring 612 is configured to elastically pressurize the test kit C inserted in the channel module 61 to be in close contact with one surface on which a blood flow detection sensor 631 is provided.

In an embodiment of the present invention, the test kit C inserted in the channel module 61 is configured to be elastically pressurized, through three elastic springs 612, toward one end and one surface on which the blood flow detection sensor 631 is provided.

The test kit C is brought into maximum contact with the blood flow detection sensor 631 by a configuration of the elastic spring 612, and accordingly, measurement errors may be minimized.

In addition, in an embodiment of the present invention, the channel module 61 is configured to have a multi-channel structure and is composed of multiple modules, and the controller 80 is configured to control the transfer actuator 32 such that the test kit C gripped and transferred by the third gripper 313 is sequentially mounted on an empty channel module 61 among the multiple channel modules 61.

In an embodiment of the present invention, FIG. 15 illustrates an example in which the channel modules 61 are configured with six channels, but the present invention is not limited thereto.

The multi-channel structure of the channel module 61 described above enables the device 1 according to the present invention to control a transfer sequence by considering a relatively long viscosity measurement time and so on, enables processing time of the device 1 to be reduced, and enables simultaneous processing of multiple blood samples.

The temperature maintenance means 62 is configured to heat or cool the test kit C mounted on the channel module 61 to enable the test kit C to maintain a set temperature.

In an embodiment of the present invention, the temperature maintenance means 62 may include a temperature sensor 621, a heater 622, a cooling fan 623, and a temperature controller (not illustrated).

The temperature sensor 621 is configured to detect temperature of the test kit C mounted on the channel module 61, and a temperature sensing unit may be attached to an inner surface of the channel module 61 but is not limited thereto.

The heater 622 is configured to heat the test kit C mounted on the channel module 61, and may be configured as a patch-type U-type heater having a flexible structure and a shape capable of wrapping both sides of the channel module 61 but is not limited thereto.

The cooling fan 623 is configured to cool the test kit C mounted on the channel module 61, and may be provided on a side surface of the channel module 61 but is not limited thereto.

The temperature controller (not illustrated) is configured to selectively operate the heater 622 or the cooling fan 623 such that the temperature detected by the temperature sensor 621 may maintain a set temperature similar to body temperature.

The temperature controller (not illustrated) may also be provided as a separate component in the viscosity measurement unit 60, or the controller 80 may also be configured to perform this function.

The viscosity measurement means 63 is configured to measure viscosity of a blood sample injected into the test kit C.

In an embodiment of the present invention, the test kit C includes a U-shaped tube C1 that allows an input blood sample to flow in an opposite direction due to a height difference when a blood sample is injected into an upper portion on one side of the test kit C.

The test kit C including the U-shaped tube C1 may utilize the "Small Blood Viscosity Measurement Kit and Its Cartridge" disclosed in Korean Patent Laid-Open Publication No. 10-21-0087898.

In an embodiment of the present invention, the viscosity measurement means 63 is provided on one surface of the channel module 61 and includes the blood flow detection sensor 631 that detects speed of a blood sample flowing to the other side of the U-shaped tube C1.

The blood flow detection sensor 631 may be configured based on a CIS sensor (Contact Image Sensor) but is not limited thereto.

Meanwhile, as described above, the test kit C is brought into maximum contact with the blood flow detection sensor 631 by a configuration of the elastic spring 612 provided in the channel module 61, and accordingly, measurement errors may be minimized.

A viscosity calculator (not illustrated) is configured to calculate viscosity of a blood sample using speed of a blood sample detected by the blood flow detection sensor 631.

That is, when viscosity of a blood sample is high, speed of a blood sample flowing from one side of the U-shaped tube C to the other side decreases, and he viscosity calculator (not illustrated) uses this phenomenon to calculate the viscosity of the blood sample based on the speed of the blood sample flowing to the other side of the U-shaped tube C.

The viscosity calculator (not illustrated) may also be provided as a separate component in the viscosity measurement means 63, or the controller 80 may also be configured to perform this function.

The kit detection sensor 64 is configured to detect whether the test kit C is mounted in the channel module 61.

The progress notification lamp 65 is configured to visually display information detected by the kit detection sensor 64 and the progress of measurement in the channel module 61.

In an embodiment of the present invention, the progress notification lamp 65 may be configured to emit light from a lower portion of the channel module 61 to an upper portion thereof and to enable a user to immediately check a state of each of the multiple channel modules 61 through the light emitted by transmitting through the test kit C.

The vibration-proof means 66 is configured to be disposed at a lower portion of the channel module 61 to attenuate vibration transferred to the channel module 61.

The vibration-proof means 66 may employ a multi-layered vibration-proof structure such as a stone plate, a square vibration-proof pad, a base plate, a circular vibration-proof pad, or any combination thereof.

The waste disposal unit 70 is configured to discard a used pipette tip B and a used test kit C.

In an embodiment of the present invention, the waste disposal unit 70 includes a waste drawer (71) that receives and accommodates the pipette tip B and test kit C to be discarded after being gripped by the second and third grippers 312 and 313 and transferred to a designated disposal position inside the housing 10 and is supported to slidingly movable back and forth inside the housing 10 such that the pipette tip B and test kit C may be taken out of the housing 10 by a user's operation.

Here, the controller 80 controls the transfer actuator 32 such that the used pipette tip B and test kit C are transferred to the waste disposal unit 70 by respectively using the second and third grippers 312 and 313.

The controller 80 is configured to check states and control operations of the housing 10, the input portion 20, the transfer unit 30, the preprocessing unit 40, the blood suction/injection unit 50, the viscosity measurement unit 60, and the waste disposal unit 70.

As illustrated in FIG. 1, the externally exposed components of the controller 80 may include a touch display 81 capable of inputting and outputting information, the power button 82 capable of turning the device 1 on and off, then emergency stop switch 83 for emergency stop, and a tower lamp 84 for warning alarms.

Meanwhile, the controller 80 may include a main body (not illustrated), such as a computer for determining a state of the device 1 and controlling the device 1.

Specifically, the controller 80 may control the door locking means 13 such that the management door 12 may be locked or opened according to a user's operation or a checked state of the device 1.

In addition, the controller 80 may control the first, second, and third drawer locking means 241, 242, and 243 such that the first, second, and third replacement drawers 211, 212, and 213 may be locked or opened according to the user's operation or the checked state of the device 1.

In addition, the controller 80 may determine a state of the device 1 by including information detected by the first, second, and third tray detection sensors 251, 252, and 253.

In addition, the controller 80 may determine a state of a device according to the progress of measurement based on information detected by the input material detection sensor 27.

In addition, the controller 80 may control the transfer actuator 32 such that the blood collection tube A gripped and transferred by the first gripper 311 is sequentially accommodated in an empty space among the multi-spaces of the preprocessing unit 40.

In addition, the controller 80 may control the transfer actuator 32 such that, when a blood sample is sucked from the blood collection tube A with the separated sealing cap A1 into the preprocessing unit 40 by using the pipette tip B, the pipette tip B is lifted and lowered according to a change in height of the blood sample contained in the blood collection tube A to enable the blood sample to be sucked at a constant depth based on a surface of the blood sample.

In addition, the controller 80 may control the blood suction/injection unit 50 such that, when a blood sample is sucked from the blood collection tube A with the separated sealing cap A1 into the preprocessing unit 40 by using the pipette tip B, the blood sample is sucked after suction and dispensing are repeated for a set number of times to enable the blood sample to be sucked in a mixed state.

In addition, the controller 80 may control the transfer actuator 32 such that the test kit C gripped and transferred by the third gripper 313 is sequentially mounted on an empty channel module 61 among the multiple channel modules 61.

In addition, the controller 80 may cause the cap separation means 43 to operate in reverse such that the sealing cap A1 separated from the used blood collection tube A is refastened to the blood collection tube A, and control the transfer actuator 32 such that the used blood collection tube A with the refastened sealing cap A1 returns to the first position P1 by using the first gripper 311, and control the transfer actuator 32 such that the used pipette tip B and test kit C to are transferred to the waste disposal unit 70 by respectively using the second and third grippers 312 and 313.

Furthermore, the controller 80 is configured to automatically measure viscosity of multiple blood samples sequentially and within a short period of time by controlling a transfer sequence of the first, second, and third grippers 311, 312, and 313 and the transfer actuator 32 in consideration of the blood mixing time in the preprocessing unit 40 having a multi-space structure, the viscosity measurement time in the viscosity measurement unit 60 having a multi-channel structure, and so on.

The transfer sequence control by the controller 80 includes, for example, controlling the input material detection sensor 27 attached to the transfer actuator 32 moved as illustrated in FIG. 15 such that, at the initial operation of the device 1, the input material detection sensor 27 may detect positions and quantities of the blood collection tube A, the pipette tip B, and the test kit C respectively input to the first, second, and third positions P1, P2, and P3 (first operation).

Thereafter, as illustrated in FIG. 12, the first gripper 311 grips the blood collection tube A located at the first position P1 (second operation).

Thereafter, as illustrated in FIG. 13, the second gripper 312 grips the pipette tip B located at the second position P2 (third operation).

Thereafter, as illustrated in FIG. 16, the blood collection tube A gripped by the first gripper 311 is controlled to be transferred to a scan position of the preprocessing unit 40 and to obtain blood collection tube information by using the scan means 41 (fourth operation).

Thereafter, as illustrated in FIG. 17, the blood collection tube A gripped by the first gripper 311 is moved to a mixing position such that the blood collection tube forceps 421 hold a body portion of the blood collection tube A, and the mixing rotation motor 422 operates to cause the blood mixing means 42 to mix the blood (fifth operation).

Thereafter, as illustrated in FIG. 14, the third gripper 313 grips the test kit C located at the third position P3 and causes the test kit C to be inserted into and mounted in the channel unit 61 of the viscosity measurement unit 60 (sixth operation).

Thereafter, as illustrated in FIG. 18, the sealing cap A1 is separated from the blood collection tube A held by the blood collection tube forceps 421 through the cap separation means 43 (seventh operation).

Thereafter, as illustrated in FIG. 19, the blood suction/injection unit 50 sucks a blood sample from the blood collection tube A with the separated sealing cap A1 into the preprocessing unit 40 and injects the blood sample into the test kit C mounted on the viscosity measurement unit 60 (eighth operation).

Thereafter, the used pipette tip B is discarded by the waste disposal unit 70 (ninth operation).

Thereafter, the cap separation means 43 is operated in reverse to the order illustrated in FIG. 18 such that the sealing cap A1 separated from the used blood collection tube A is refastened to the blood collection tube A, and the used blood collection tube A with the refastened sealing cap A1 returns to the first position P1 by using the first gripper 311 (tenth operation).

Thereafter, the second through tenth operations are repeated to measure viscosity of another blood sample.

Meanwhile, in an embodiment of the present invention, the second and fourth operations may be performed between the second through tenth operations to utilize the preprocessing unit 40 having a multi-space structure, and the test kit C, which is used because viscosity measurement is completed during the second to tenth operations, may be discarded by the waste disposal unit 70 to utilize the viscosity measurement unit 60 having a multi-channel structure.

The automatic blood viscosity measurement device described above and illustrated in the drawings is merely an example, for implementing the present invention and should not be construed as limiting the technical idea of the present invention. The scope of protection of the present invention is defined solely by the matters set forth in the following claims, and improvements and modifications made without departing from the idea of the present invention are deemed to fall within the scope of protection of the present invention, provided that such improvements and modifications are obvious to those skilled in the art.

## Claims

1. A blood viscosity measurement module for an automatic blood viscosity measurement device, the module comprising:
a viscosity measurement unit configured to measure viscosity of a blood sample injected into a test kit gripped by a gripper and transferred by a transfer actuator; and
a controller configured to check a state of the viscosity measurement unit and control an operation of the viscosity measurement unit,
wherein the viscosity measurement unit includes:
a channel module onto which the test kit gripped and transferred by the gripper is inserted in a vertical direction and on which the test kit is mounted;
a temperature maintenance means including a heater, for heating or cooling the test kit mounted on the channel module, such that the test kit maintains a set temperature; and
a viscosity measurement means including a blood flow detection sensor for measuring the viscosity of the blood sample injected into the test kit.

2. The blood viscosity measurement module of claim 1, wherein
the channel module includes a plurality of channel modules to have a multi-channel structure and the test kit includes a plurality of test kits, and
the controller controls the transfer actuator such that the plurality of test kits gripped and transferred by the gripper are sequentially mounted on empty channel modules among the plurality of channel modules.

3. The blood viscosity measurement module of claim 1, wherein the temperature maintenance means includes:
a temperature sensor configured to detect temperature of the test kit mounted on the channel module;
the heater configured to heat the test kit mounted on the channel module;
a cooling fan configured to cool the test kit mounted on the channel module; and
a temperature controller configured to selectively operate one of the heater and the cooling fan such that the temperature detected by the temperature sensor maintains the set temperature.

4. The blood viscosity measurement module of claim 1, wherein
the test kit includes a U-shaped tube such that, when a blood sample is injected into an upper portion on one side of the U-shaped tube, the injected blood sample flows to another side of the U-shaped tube due to a height difference of the injected blood sample, and
the blood flow detection sensor of the viscosity measurement means is disposed on one surface of the channel module and configured to detect a speed of the blood sample flowing to the another side of the U-shaped tube; and
a viscosity calculator configured to calculate the viscosity of the blood sample using the speed of the blood sample detected by the blood flow detection sensor.

5. The blood viscosity measurement module of claim 4, wherein
the channel module includes an elastic spring configured to elastically pressurize the inserted test kit in a direction in which the test kit comes into contact with the one surface on which the blood flow detection sensor is disposed.

6. The blood viscosity measurement module of claim 1, wherein the viscosity measurement unit further includes:
a kit detection sensor configured to detect whether the test kit is mounted on the channel module; and
a progress notification lamp configured to visually display information detected by the kit detection sensor and progress of measurement in the channel module.

7. The blood viscosity measurement module of claim 1, wherein
the viscosity measurement unit further includes a vibration-proof means including a pad and disposed at a lower portion of the channel module to attenuate vibration transmitted to the channel module.
